(19) **Europäisches Patentamt European Patent Office Office européen des brevets**

(11) **EP 3 973 878 B1**

(12) **EUROPEAN PATENT SPECIFICATION**

(45) Date of publication and mention
of the grant of the patent:
**11.12.2024  Bulletin 2024/50**

(21) Application number: **20809402.9**

(22) Date of filing: **26.02.2020**

(51) International Patent Classification (IPC):
*A61B 6/00* *(2024.01)*          *A61B 6/50* *(2024.01)*
*A61B 6/03* *(2006.01)*          *G06N 20/00* *(2019.01)*
*A61B 6/51* *(2024.01)*

(52) Cooperative Patent Classification (CPC):
**A61B 6/51; A61B 6/506; A61B 6/5217;
A61B 6/5223; G06N 20/00;** A61B 6/032;
G06N 3/045

(86) International application number:
**PCT/KR2020/002757**

(87) International publication number:
**WO 2020/235784 (26.11.2020 Gazette 2020/48)**

(54) **NERVE DETECTION METHOD AND DEVICE**

VERFAHREN UND VORRICHTUNG ZUR NERVENERKENNUNG

PROCÉDÉ ET DISPOSITIF DE DÉTECTION DE NERF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.05.2019  KR 20190060042
26.06.2019  KR 20190076079**

(43) Date of publication of application:
**30.03.2022  Bulletin 2022/13**

(73) Proprietor: **DIO Corporation
Busan 48058 (KR)**

(72) Inventors:
• **KIM, Jin Cheol
Busan 48058 (KR)**

• **KIM, Jin Baek
Busan 48058 (KR)**

(74) Representative: **Maiwald GmbH
Elisenhof
Elisenstraße 3
80335 München (DE)**

(56) References cited:
WO-A1-2019/002631      WO-A1-2019/002631
CN-A- 109 875 683      KR-A- 20170 091 847
KR-A- 20200 134 512      KR-A- 20210 000 855
KR-B1- 101 839 789      KR-B1- 101 908 963
KR-B1- 101 974 636      KR-B1- 101 974 636

## Description

## BACKGROUND

### 1. Field of the Invention

The present invention relates to a nerve detection method and device, and more specifically to a method and device for detecting a nerve based on the anatomical characteristics of nerve distribution.

### 2. Discussion of Related Art

In general, implant refers to a substitute that can replace human tissue when the original human tissue is lost, and particularly, in dentistry, it refers to implanting an artificially made tooth into the location of an actual tooth using prostheses including fixtures, abutments and crowns.

In dentistry, implant surgery is performed by forming a perforation in the alveolar bone and placing a fixture in the perforation, and when the fixture is fused to the alveolar bone, an abutment and a crown are coupled to the fixture.

In order to prevent problems such as nerve damage and the like, such implant surgery determines the placement position, angle, depth and the like of the implant based on cone beam computed tomography (CBCT) images prior to the implant surgery, thereby confirming the positions of the bone tissues such as teeth and the alveolar bone, neural tubes and the like in advance.

In particular, drilling for alveolar bone perforation during implant surgery requires maintaining a safe distance between the fixture and the inferior alveolar nerve, which is difficult due to various nerve paths and conditions for each patient. In addition, conventionally, the inferior alveolar nerve has been detected using the panoramic image of tomography images, but there exists an opaque and unclear inferior alveolar nerve in the panoramic image, which may cause damage to the nerve during implant surgery. WO2019002631A1 discloses a nerve detection apparatus.

## SUMMARY OF THE INVENTION

In order to solve such conventional problems, it is an object of the present invention to provide a nerve detection method and device capable of automatically detecting nerves faster and more accurately by detecting nerves based on the anatomical characteristics of nerve distribution, and accurately identifying the location and condition of different neural tubes for each patient to safely perform implant surgery.

In addition, it is an object of the present invention to provide a method and device for correcting a nerve location capable of simply and more accurately correcting a nerve location detected by various nerve location detection techniques.

The technical problems to be achieved in the present invention are not limited to the technical problems mentioned above, and other technical problems that are not mentioned will be clearly understood by those of ordinary skill in the art to which the present invention pertains from the description below.

In order to solve the aforementioned problems, the present invention provides a method for detecting a nerve, including acquiring a learning image including a plurality of slices arranged in a coronal plane direction, generating learning data in which a nerve and mental tubercle and/or a mandibular angle, serving as a reference for detecting the nerve, are set in the learning image, and learning the learning data so as to generate a learning model, inputting a target image into the learning model, and detecting a nerve from the target image on the basis of the learning data.

Herein, the detecting a nerve from the target image may include detecting the mental tubercle and mandibular angle from the target image, setting a section between the detected mental tubercle and mandibular angle as a nerve effective section, and detecting the nerve by searching the nerve effective section.

In addition, the generating learning data is generating the learning data in which at least one of the alveolar bone, the mental nerve and the inferior alveolar nerve is set in the learning image.

In addition, the detecting the nerve from the target image is detecting the alveolar bone, the mental nerve and the inferior alveolar nerve from the target image.

In addition, the detecting the inferior alveolar nerve is detecting the inferior alveolar nerve using the detected alveolar bone.

In addition, the detecting the mental tubercle from the target image is detecting the mental tubercle by setting a first search starting point based on the statistics of the learning image, and searching in the outer direction of the target image from the first search starting point.

In addition, the detecting the mandibular angle from the target image is detecting the mandibular angle by setting a second search starting point based on the statistics of the learning image, and searching in the outer direction of the target image from the second search starting point.

In addition, the detecting a nerve from the target image may include setting a target region expected to include the mental tubercle, the mandibular angle and the nerve which are detection targets in the target image, calculating location and size information of the target region, and calculating a probability value that the detection target is included in the target region.

In addition, the detecting a nerve from the target image may further include detecting the target region in which the probability value is more than or equal to a reference value as a detection region including the detection target.

In addition, the detecting a nerve from the target image may further include extracting coordinates of a region including the nerve from the detection region, and detecting the nerve based on the coordinates.

In addition, the method for detecting a nerve according to the present invention may further include acquiring a curved planar reformation (CPR) image based on the nerve location, respectively displaying a nerve location and a reference line on the curved planar reformation image, and correcting the nerve location on the curved planar reformation image based on the reference line.

Herein, the coordinate values of the reference line may include at least one of the coordinate values of the nerve location.

In addition, the reference line may be a line passing through a part of a nerve region displayed in the curved planar reformation image.

In addition, the present invention provides a device for detecting a nerve, including an Image collector for acquiring a learning image including a plurality of slices arranged in a coronal plane direction, a Learner for generating learning data in which a nerve and mental tubercle and/or a mandibular angle, serving as a reference for detecting the nerve, are set in the learning image, and learning the learning data so as to generate a learning model, and a Nerve detector for detecting a nerve from the target image on the basis of the learning data.

In addition, the device for detecting a nerve according to the present invention may further include a Corrector for acquiring a curved planar reformation (CPR) image based on the nerve location, displaying a reference line on the curved planar reformation image, and correcting the nerve location based on the reference line.

According to the present invention, it is possible to automatically detect nerves faster and more accurately by detecting the nerves based on the anatomical characteristics of nerve distribution, and through this, there is an effect that it is possible to accurately identify the location and condition of different neural tubes for each patient to safely perform implant surgery.

In addition, according to the present invention, there is an effect that it is possible to simply and more accurately correct a nerve location detected by various nerve location detection techniques.

The effects that can be obtained in the present invention are not limited to the above-mentioned effects, and other effects that are not mentioned will be clearly understood by those of ordinary skill in the art to which the present invention pertains from the following description.

## BRIEF DESCRIPTION OF THE DRAWINGS

FIG. 1 is a schematic block diagram of the electronic device for detecting a nerve from a target image according to an exemplary embodiment of the present invention.

FIG. 2 is a detailed block diagram of the Controller of FIG. 1.

FIG. 3 is a flowchart of the method for detecting a nerve from a target image according to an exemplary embodiment of the present invention.

FIG. 4 is a diagram illustrating the cross-sectional images of teeth that vary depending on the direction of the patient's head.

FIG. 5 is a diagram respectively illustrating detection targets on tooth cross-sectional images in a coronal plane direction.

FIG. 6 is a diagram for describing the method of generating learning data according to an exemplary embodiment of the present invention.

FIG. 7 is a flowchart of detecting a nerve from the target image of FIG. 6.

FIG. 8 is a diagram for describing the step of detecting a nerve from the target image of FIG. 6.

FIG. 9 is a detailed flowchart of detecting a nerve from the target image of FIG. 7.

FIG. 10 is a flowchart of correcting a nerve location according to an exemplary embodiment of the present invention.

FIG. 11 is a diagram illustrating an example of a 3D image indicating nerve locations.

FIG. 12 is a diagram illustrating an example of the curved planar reformation image obtained in FIG. 11.

FIG. 13 is a diagram illustrating a process of acquiring a curved planar reformation image.

FIG. 14 shows an example in which a nerve location is displayed on the curved planar reformation image of FIG. 12.

FIG. 15 is a diagram illustrating an example in which a nerve location and reference lines are displayed together in the curved planar reformation image of FIG. 12.

FIG. 16 is a diagram illustrating an example of correcting a nerve location displayed in the curved planar reformation image of FIG. 12 along reference lines.

FIG. 17 is a diagram illustrating an example of a curved planar reformation image after correction is performed according to FIG. 16.

FIGS. 18 to 21 are diagrams illustrating various examples of a user interface (UI) that displays curved planar reformation images.

FIG. 22 is a diagram illustrating various ranges of curved planar reformation images.

FIG. 23 is a diagram showing a comparison between the conventional correction method and the method for correcting a nerve location according to an exemplary embodiment of the present invention.

## DETAILED DESCRIPTION OF EXEMPLE EMBODIMENTS

Hereinafter, preferred exemplary embodiments according to the present invention will be described in detail with reference to the accompanying drawings. The detailed description disclosed below in conjunction with the accompanying drawings is intended to describe exemplary embodiments of the present invention and is not intended to represent the only exemplary embodiments in which the present invention may be practiced. In order to clearly describe the present invention in the drawings, parts irrelevant to the description may be omitted, and the same reference numerals may be used for the same or similar components throughout the specification.

In an exemplary embodiment of the present invention, expressions such as "or", "at least one" and the like may indicate one of the words listed together, or a combination of two or more. For example, "A or B" and "at least one of A and B" may include only one of A or B, or both A and B.

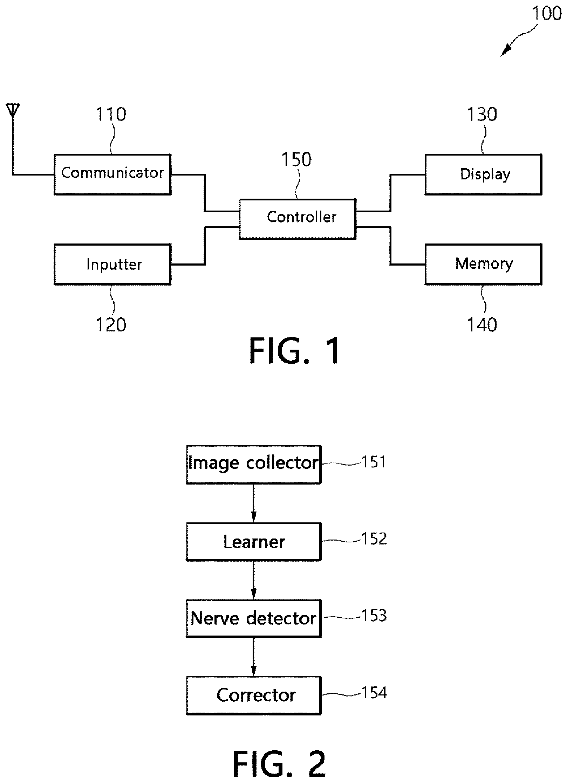

FIG. 1 is a schematic block diagram of the electronic device for detecting a nerve from a target image according to an exemplary embodiment of the present invention, and FIG. 2 is a detailed block diagram of the Controller of FIG. 1.

Referring to FIG. 1, the electronic device 100 according to the present invention may include a communicator 110, an Iputter 120, a Display 130, a memory 140 and a Controller 150.

The communicator 110 communicates with external devices such as an image acquisition device (not illustrated), a server (not illustrated) and the like. To this end, the communicator 110 may perform wireless communication such as 5th generation communication (5G), long term evolution-advanced (LTE-A), long term evolution (LTE), Bluetooth, Bluetooth low energy (BLE), near-field communication (NFC) and the like, or wired communication such as cable communication and the like.

The Iputter 120 generates input data in response to a user's input of the electronic device 100. The Iputter 120 includes at least one input means. The Iputter 120 may include a keyboard, a keypad, a dome switch, a touch panel, a touch key, a mouse, a menu button and the like.

The Display 130 displays display data according to an operation of the electronic device 100. The Display 130 includes a liquid crystal display (LCD), a light-emitting diode (LED) display, an organic light-emitting diode (OLED) display, a micro-electro mechanical system (MEMS) display and an electronic paper display. The Display 130 may be combined with the Iputter 120 to be implemented as a touch screen.

The memory 140 stores operation programs of the electronic device 100. In addition, the memory 140 may store an algorithm related to a convolutional neural network (CNN) such as U-Net, RCNN and the like. The memory 140 may store a plurality of learning images received from an image acquisition device or the like.

The Controller 150 learns the learning images to generate learning data, and detects a nerve from the target image based on the learning data. In particular, based on the anatomical characteristic that the nerve is located between the mental tubercle and mandibular angle, the Controller 150 has an advantage of detecting the nerve more quickly and accurately by first detecting the mental tubercle and mandibular angle, and then detecting the nerve located between the mental tubercle and the mandibular angle.

Referring to FIG. 2, the Controller 150 may include an Image collector 151, a Learner 152 and a Nerve detector 153.

As such, the Controller 150 is characterized in that it generates learning data based on the tooth cross-sectional images in a coronal plane direction, which clearly show the anatomical sites such as the mental tubercle, the mental nerve, the inferior alveolar nerve, the alveolar bone, the mandibular angle and the like, and clearly show the distinctions thereof, thereby detecting a nerve based thereon.

The Image collector 151 receives a 3D image from an external image acquisition device and converts the same into a plurality of 2D cross-sectional images (hereinafter, slices) arranged in a coronal plane direction. That is, the Image collector 151 acquires a learning image including a plurality of slices arranged in a coronal plane direction.

The Learner 152 generates learning data in which nerves (the mental nerve and the inferior alveolar nerve) and at least one of the mental tubercle and the mandibular angle as a reference for detecting the nerves, and the alveolar bone that helps to detect the inferior alveolar nerve are set in a plurality of learning images acquired by the Image collector 151, and generates a learning model by learning the learning data. Herein, the Learner 152 may learn the learning images using an algorithm related to a convolutional neural network (CNN) such as U-Net, RCNN and the like, which are stored in the memory 140.

The Nerve detector 153 inputs a target image into the learning model generated by the Learner 152 to detect a nerve from the target image based on the learning data. Herein, the target image may be a 3D image including a plurality of slices (2D cross-sectional images) arranged in a coronal plane direction of the patient.

Specifically, the Nerve detector 153 detects the mental tubercle and mandibular angle from the target image based on the learning data, sets a section between the detected mental tubercle and mandibular angle as a nerve effective section, and searches the set nerve effective section to detect nerves (the mental nerve and the inferior alveolar nerve). Herein, the Nerve detector 153 may detect the alveolar bone and use the same to detect the inferior alveolar nerve. That is, since the inferior alveolar nerve is located inside the alveolar bone, detection accuracy may be improved by detecting the alveolar bone first and then detecting the inferior alveolar nerve in the detected alveolar bone.

Meanwhile, the meaning that the Nerve detector 153 detects the mental tubercle, the mandibular angle, the alveolar bone and the nerve may be interpreted as including the meaning of detecting a slice including these detection targets.

The Nerve detector 153 may set a first search starting point based on the statistics of a plurality of learning images, and detect the mental tubercle by searching in the outer direction of the target image from the set first search starting point.

Likewise, the Nerve detector 153 may set a second search starting point based on the statistics of a plurality of learning images, and detect the mandibular angle by searching in the outer direction of the target image from the second search starting point.

Herein, the statistics of a plurality of learning images is a value (e.g., 0 to 1) obtained by normalizing the position of anatomical sites included in each image, focusing on the difference in resolution of the captured images for each image acquisition device. Statistically, there is little likelihood of finding the mental tubercle at the first search starting point (e.g., 0.323) or more, and little likelihood of finding the mandibular angle at the second search starting point (e.g., 0.715) or less. Therefore, if the mental tubercle and mandibular angle are searched based on the first search starting point and the second search starting point, the mental tubercle and the mandibular angle may be searched more quickly and accurately.

The Nerve detector 153 sets a target region that is expected to include the mental tubercle, the mandibular angle and nerves to be detected in the target image. Then, position and size information of the target region is calculated, and a probability value in which the detection target is included in the target region is calculated. In addition, a target region having a calculated probability value which is more than or equal to a reference value is detected as a detection region including the detection target, and the detection target is labeled such that it is displayed in a slice including the corresponding region.

The Nerve detector 153 extracts coordinates (e.g., center coordinates) of a region including a nerve in the detection region, and detects a nerve based on the extracted coordinates (e.g., center coordinates). That is, by collecting coordinates (e.g., center coordinates) of a region including a nerve from a plurality of slices, a neural tube may be finally extracted.

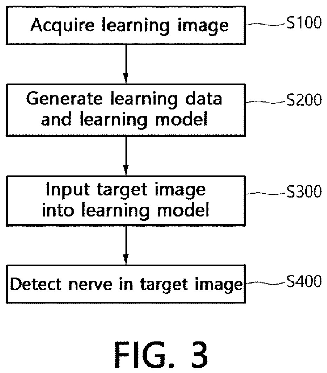

FIG. 3 is a flowchart of the method for detecting a nerve from a target image according to an exemplary embodiment of the present invention.

Referring to FIG. 3, the nerve detection method according to an exemplary embodiment of the present invention may be configured by including the steps of acquiring a learning image S 100, generating learning data and a learning model S200, inputting a target image into the learning model S300 and detecting a nerve from the target image S400.

In the step of acquiring a learning image S 100, a 3D image is received from an external image acquisition device and converted into a plurality of 2D cross-sectional images (hereinafter, slices) arranged in a coronal plane direction. That is, a learning image including a plurality of slices arranged in a coronal plane direction is acquired.

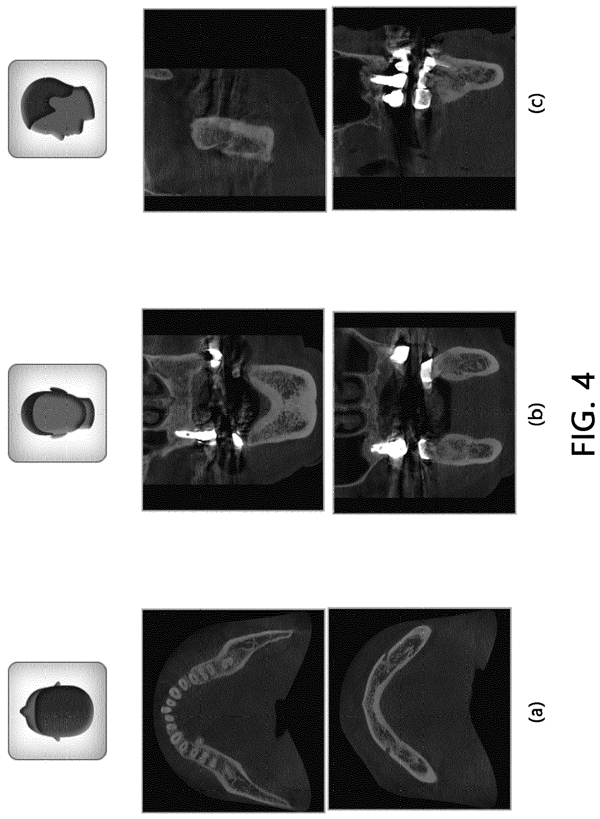

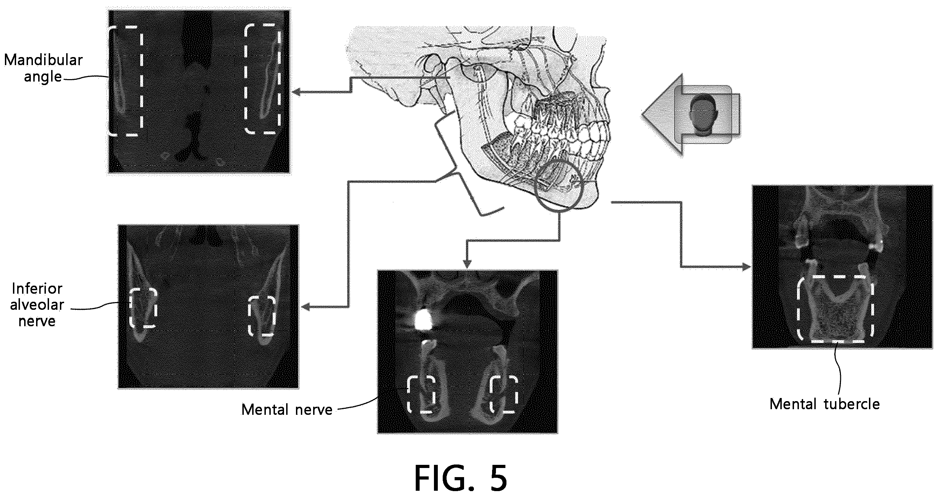

FIG. 4 is a diagram illustrating the cross-sectional images of teeth that vary depending on the direction of the patient's head, and FIG. 5 is a diagram respectively illustrating detection targets on tooth cross-sectional images in a coronal direction.

Referring to FIG. 4, the direction of the patient's head may be divided into an axial plane direction (a), a coronal plane direction (b) and a sagittal plane direction (c), and it can be confirmed that the anatomical sites of teeth appearing in the tooth cross-section images (slices) are different depending on the direction of the head.

Referring to FIGS. 4 and 5, the nerve detection method according to an exemplary embodiment of the present invention is characterized in that it generates learning data based on the tooth cross-sectional images in a coronal plane direction (b), which clearly show the anatomical sites such as the mental tubercle, the mental nerve, the inferior alveolar nerve, the alveolar bone, the mandibular angle and the like, and clearly show the distinctions thereof, thereby detecting a nerve based thereon.

For reference, referring to FIG. 5, it can be confirmed that the inferior alveolar nerve is located inside the alveolar bone, and the mental nerve is not located inside the alveolar bone, but it is in a form pierced outward.

The step of generating learning data and a learning model S200 generates learning data in which nerves (the mental nerve and the inferior alveolar nerve) and at least one of the mental tubercle and the mandibular angle as a reference for detecting the nerves, and the alveolar bone that helps to detect the inferior alveolar nerve are set in a plurality of acquired learning images, and generates a learning model by learning the learning data.

Herein, a learning model may be generated by learning the learning data using an algorithm related to a convolutional neural network (CNN) such as U-Net, RCNN and the like, which are stored in the memory 140.

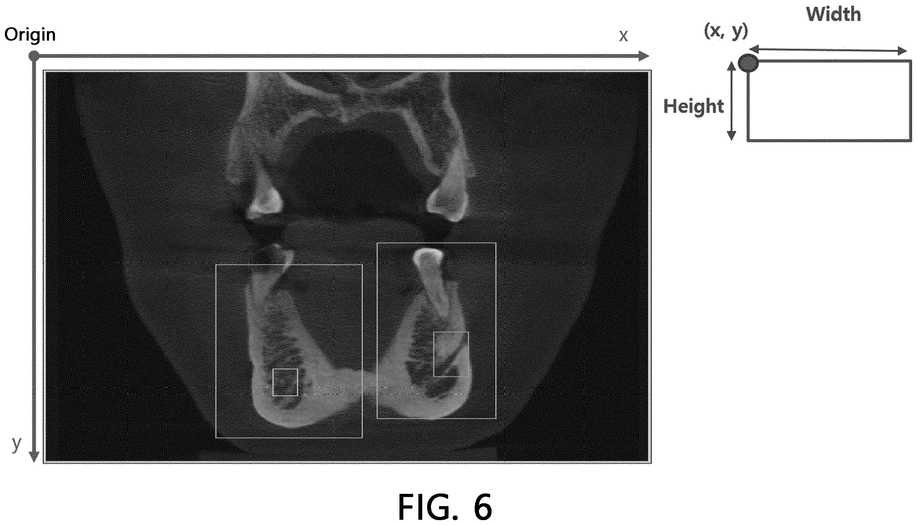

FIG. 6 is a diagram for describing the method of generating learning data according to an exemplary embodiment of the present invention.

Referring to FIG. 6, the learning data may set location and size information for a region (e.g., a box) including the mental tubercle, the mandibular angle and the alveolar bone and nerves (learning targets) for each of a plurality of slices included in the learning image. Specifically, the x-axis and y-axis positions of the upper left of the box may be set as the position coordinates of the box with the upper left of the learning image as the origin, and the width and height of the box may be set based on the set position coordinates.

Meanwhile, the method of setting the location and size information of the learning targets in the learning image as described above may be applied as it is to the method of setting the position and size information of the detection targets in the target images to be described below.

After inputting the target image into the learning model S300, the step of detecting a nerve from the target image S400 detects the nerve in the target image based on the learning data. Herein, the target image may be a 3D image including a plurality of slices (2D cross-sectional images) arranged in a coronal plane direction of the patient.

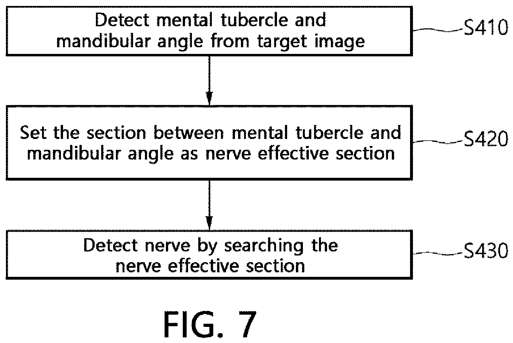

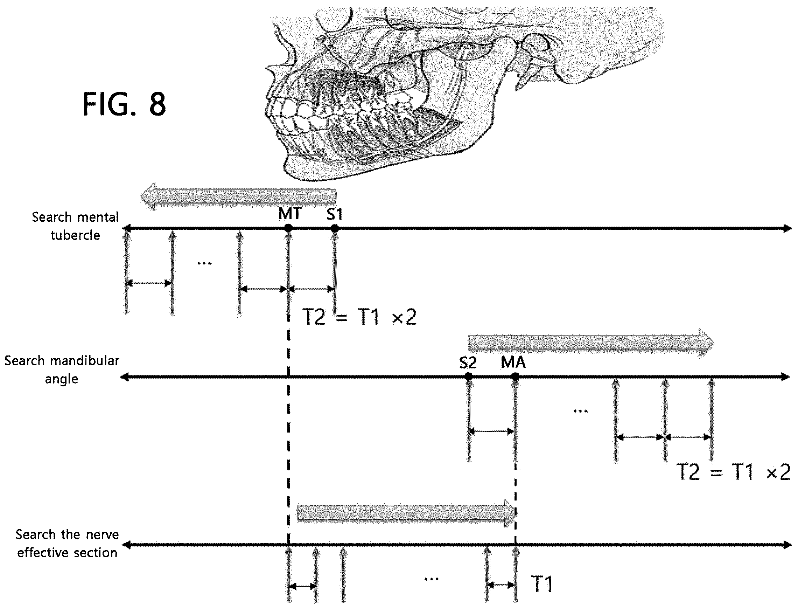

FIG. 7 is a flowchart of detecting a nerve from the target image of FIG. 6, and FIG. 8 is a diagram for describing the step of detecting a nerve from the target image of FIG. 6.

Referring to FIG. 7, the step of detecting a nerve from the target image S400 detects the mental tubercle and the mandibular angle from the target image based on the learning data S410, sets a section between the detected mental tubercle and mandibular angle as a nerve effective section S420, and detects nerves (the mental nerve and the inferior alveolar nerve) by searching the set nerve effective section S430.

Herein, the detected alveolar bone may be used to detect the inferior alveolar nerve. That is, since the inferior alveolar nerve is located inside the alveolar bone, detection accuracy may be improved by detecting the alveolar bone first and then detecting the inferior alveolar nerve from the detected alveolar bone.

Meanwhile, the meaning of detecting the mental tubercle, the mandibular angle and the alveolar bone and nerves may be interpreted as including the meaning of detecting a slice including these detection targets.

Referring to FIG. 8, the step of detecting a nerve from the target image S400 may set a first search starting point S1 based on the statistics of a plurality of learning images, and detect the mental tubercle (MT) by searching in the outer direction of the target image at the set first search starting point S1.

Likewise, it is possible to set a second search starting point S2 based on the statistics of a plurality of learning images, and detect the mandibular angle (MA) by searching in the outer direction of the target image at the second search starting point S2.

Herein, a section between the detected mental tubercle (MT) and mandibular angle (MA) may be set as a nerve effective section, and nerves (the mental nerve and the inferior alveolar nerve) may be detected by searching the set nerve effective section.

Herein, the statistics of a plurality of learning images is a value (e.g., 0 to 1) obtained by normalizing the position of anatomical sites included in each image, focusing on the difference in resolution of the captured images for each image acquisition device. Statistically, there is little likelihood of finding the mental tubercle at the first search starting point (e.g., 0.323) or more, and little likelihood of finding the mandibular angle at the second search starting point (e.g., 0.715) or less. Therefore, if the mental tubercle and the mandibular angle are searched based on the first search starting point and the second search starting point, the mental tubercle and mandibular angle may be searched more quickly and accurately.

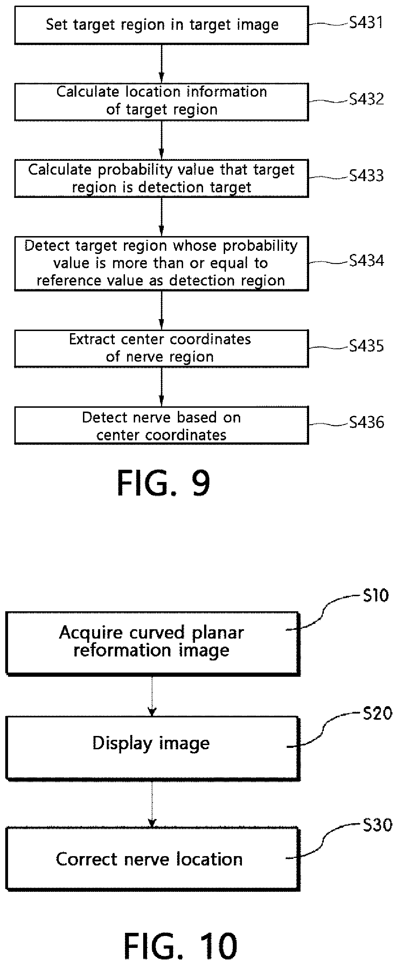

FIG. 9 is a detailed flowchart of detecting a nerve from the target image of FIG. 7.

Referring to FIG. 9, the step of detecting a nerve from a target image S400 sets a target region that is expected to include the mental tubercle, the mandibular angle and nerves, which are detection targets in the corresponding image based on the learning data S431. Then, position and size information of the target region is calculated S432, and a probability value in which the detection target is included in the target region is calculated S433. In addition, a target region having a calculated probability value which is more than or equal to a reference value may be detected as a detection region including the detection target S434, and the detection target may be labeled such that it is displayed in a slice including the corresponding region.

In addition, it extracts coordinates (e.g., center coordinates) of a region including a nerve in the detection region, and detects a nerve based on the extracted coordinates (e.g., center coordinates). That is, by collecting coordinates (e.g., center coordinates) of a region including a nerve from a plurality of slices, a neural tube may be finally extracted.

As described above, the nerve detection method according to an exemplary embodiment of the present invention detects nerves based on the anatomical characteristics of nerve distribution such that the nerves may be automatically detected more quickly and accurately, and through this, by accurately identifying the location and condition of neural tubes which are different for each patient, it is possible to safely perform implant surgery.

Referring to FIG. 2, the nerve detection device according to an exemplary embodiment of the present invention may further include a Corrector 154.

Hereinafter, the method for correcting a nerve location according to an exemplary embodiment of the present invention performed by the Corrector 154 will be described.

FIG. 10 is a flowchart of correcting a nerve location according to an exemplary embodiment of the present invention.

Referring to FIG. 10, the method for correcting a nerve location according to an exemplary embodiment of the present invention is a method of correcting a nerve location previously detected in a 3D image, and may include S500 to S700. Hereinafter, for convenience of explanation, a 3D image is an image related to a tooth and its surrounding skeletal structure, and the drawings will be illustrated and described assuming that the nerve which is previously detected therefrom is the alveolar nerve (particularly, the inferior alveolar nerve), but the present invention is not limited thereto.

S500 is an image acquisition step, and it is a step of acquiring a curved planar reformation (CPR) image based on a previously detected nerve location.

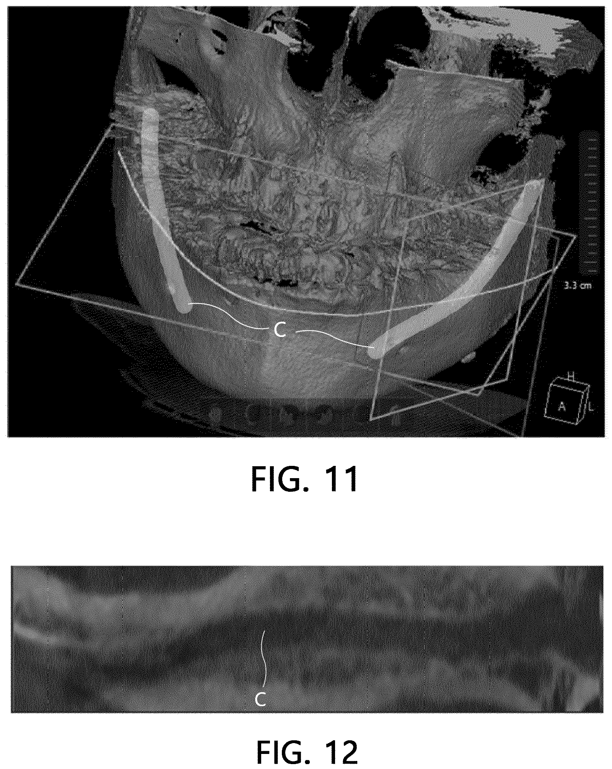

FIG. 11 illustrates an example of a 3D image indicating nerve locations. In addition, FIG. 12 illustrates an example of the curved planar reformation image obtained in FIG. 11.

Referring to FIG. 11, the nerve location of the alveolar nerve (C) may be displayed in a separate color for the region in a 3D image. As illustrated in FIG. 12, curved planar reformation images may be obtained through a process of acquiring curved planar reformation images to be described below based on the nerve location.

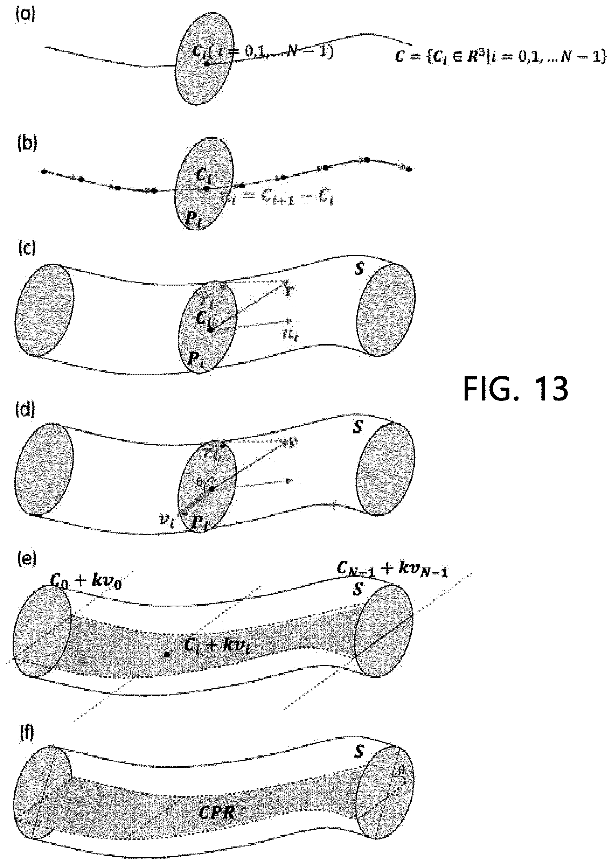

FIG. 13 illustrates a process of acquiring a curved planar reformation image. Referring to FIG. 13, the process of acquiring a curved planar reformation image will be described as follows.

First, the nerve location of the alveolar nerve (C) in the 3D image of a tooth and its surrounding skeletal structure may include the N center coordinate values of C ($C_i$) (where i = 0, 1, ... N-1) (refer to FIG. 13(a)). That is, $C_i$ is coordinate values constituting C, which are N coordinate values for points of the center curve. Accordingly, C may be formed as a spatial area expanded by a predetermined length (R) from the center line where each $C_i$ is connected. That is, it can be seen that C = {$C_i \in R^3$ | i=0, 1, ... N-1}.

In this case, planes ($P_i$) perpendicular to the normal vectors ($n_i$) and each $n_i$ at each $C_i$ may be derived (refer to FIG. 13(b) below). That is, $n_i$ is vectors representing the tangent direction at each $C_i$ and has coordinate values of $n_i = <n_x, n_y, n_z>$, and may be derived through the calculation of $C_{i+i}$ - $C_i$.

In addition, the orthogonal projection vector ($\widehat{r_i}$) according to the reference vector (r) at each $C_i$ may be derived (refer to FIG. 5(c)). In other words, $\widehat{r_i}$ is a vector in which r is projected onto $P_i$. In addition, a plane (hereinafter, referred to as an "orthogonal plane") having a region having $C_i$ as a center point and $\widehat{r_i}$ as a radius on a vertical plane may be derived from $P_i$. In this case, r is a parameter value for the range for calculating the curved planar reformation image, and it may be preset or input.

When these orthogonal projection planes are collected, a sample area (S), which is a curved area for a curved planar reformation image, may be obtained (refer to FIG. 13(f)). In this case, the curved planar reformation (CPR) image may be finally obtained according to the cross-sectional direction (Θ) at each $P_i$ in S (refer to FIGS. 13(d) and 13(e)). That is, θ is a parameter value for the angle of CPR, and it may be preset or input.

That is, the CPR image is composed of a curved plane constituting C, and may be obtained by $C_i$, r and θ. In particular, $n_i \cdot v_i = 0$, and

$$\widehat{r_i} \cdot \mathrm{vi} = |\widehat{r_i}| \, |\mathrm{vi}| \cos\theta$$

. Accordingly, coordinate values constituting CPR are equal to $C_i + kV_i$. In this case, k is a y-axis range-specified scalar value (real number, $K \in R^3$). Accordingly, the resolution (the number of samplings) of the CPR image may be determined by the number of $C_i$ (N value) and the k value.

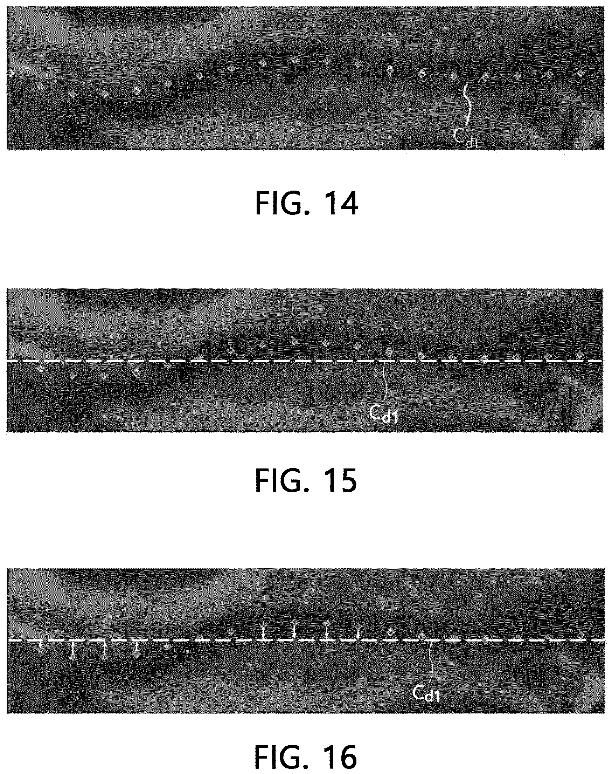

FIG. 14 shows an example in which nerve locations are displayed on the curved planar reformation image of FIG. 12, and FIG. 15 is a diagram illustrating an example in which nerve locations and reference lines are displayed together in the curved planar reformation image of FIG. 12.

Afterwards, as S600 is a display step, it is a step of displaying the CPR image acquired in S500, as illustrated in FIG. 12. In particular, in S600, as illustrated in FIGS. 14 and 15, a nerve location (rhombus) and a reference line (dotted line) may be displayed together on a CPR image. In this case, points indicating $C_i$ may be displayed as the nerve location.

In particular, the reference line is a guide line for correcting the nerve location. In other words, the alveolar nerve region before correction ($C_{d1}$) (the black area between the upper and lower white areas) and the line of the nerve location (the line connecting the rhombus) displayed on the CPR image must be displayed in a straight line due to the characteristics of the alveolar nerve. However, referring to FIG. 14, the line of $C_{d1}$ and its nerve location appears in a curved form of '~.' This indicates that a preset nerve location has been established.

Accordingly, the reference line provides a reference for correcting the alveolar nerve region ($C_{d1}$) before correction, which is displayed on the CPR image, on the CPR based on the nerve location that is incorrectly set as described above. That is, the shape of the reference line displayed on the CPR may vary depending on the type and shape of the target nerve, and it may be a straight line or curved shape. In particular, when the target nerve is the alveolar nerve, the reference line may be displayed on the CPR image in a straight line by reflecting the shape of the alveolar nerve.

However, in most cases, since only a part of the nerve location will be corrected, it may be preferable that the coordinate values of the reference line include at least one of the coordinate values of the nerve location. That is, the reference line may be a line passing through a part of the nerve region displayed on the CPR image. Since various types of the reference line are stored, the user may select the reference line through the Iputter 120, and a type designated as a default may be presented.

Meanwhile, the nerve location and the reference line may be displayed on the CPR image in different types of lines, shapes, thicknesses, colors and the like to distinguish the same.

FIG. 16 illustrates an example of correcting nerve locations displayed in the curved planar reformation image of FIG. 12 along reference lines.

Afterwards, as S700 is a correction step, it is a step of correcting a nerve location, which is displayed on the CPR image, on the CPR image based on the reference line displayed on the CPR image. That is, the point or line of the nerve location displayed on the CPR image may be changed on the CPR image (a part to be changed in FIG. 16 is indicated by an arrow) so as to correspond to the reference line. Accordingly, it is possible to correct by changing the coordinate values of the nerve location of the changed part to the coordinate values of the corresponding part in real time. In this case, the selection of the part to be changed among the nerve locations displayed on the CPR image and the degree of change may be input from the user through the Iputter 120 and performed according to the input data or by various algorithms such as machine learning and the like.

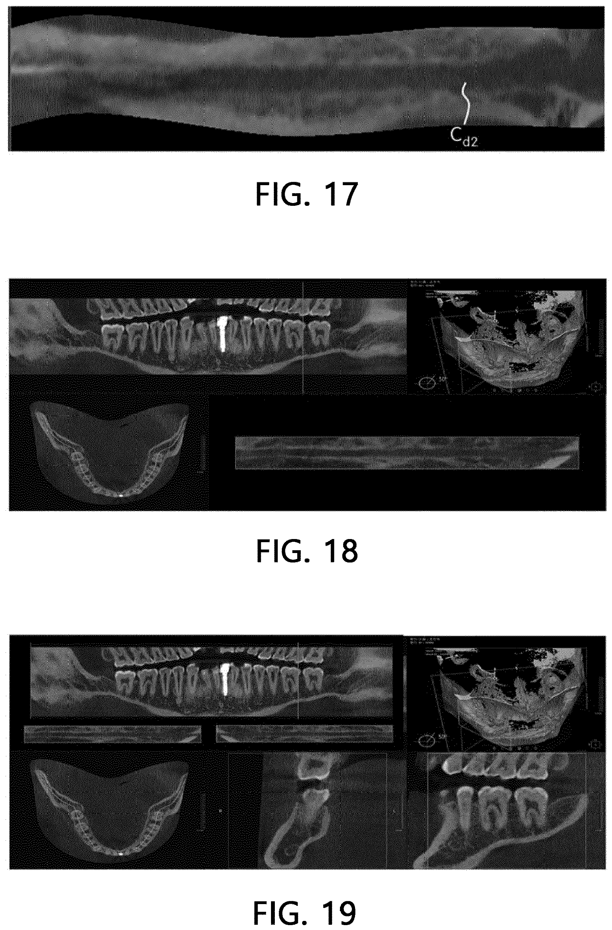

FIG. 17 illustrates an example of a curved planar reformation image after correction is performed according to FIG. 16.

According to S700, if the nerve location displayed on the CPR image is corrected on the CPR image based on the reference line, as illustrated in FIG. 17, the alveolar nerve region ($C_{d2}$) after correction displayed on the CPR image (the black area between the upper and lower white areas) is displayed almost in a straight line. This is $C_{d2}$ corresponding to the alveolar nerve, which is the target nerve, indicating that $C_{d1}$ has been properly corrected.

Meanwhile, S500 to S700 may be repeatedly performed. In this case, by updating the previously detected nerve location to the nerve location corrected in previous S700, S500 to S700 may be repeatedly performed based thereon. That is, if the nerve location is corrected by performing S500 to S700 as previous steps, when performing S500 to S700 again later, S500 to S700 may be performed based on the nerve location corrected in the previous step. Through such repetitive performance, the nerve location may be corrected more accurately.

In particular, when repeatedly performed, a CPR image having an angle ($\theta_2$) different from the angle ($\theta_1$) of the previously acquired CPR image may be acquired in S500. However, the selection of $\theta_2$ may be selected according to a predetermined angle change value selected or preset according to the input data received from the user through the Iputter 120 within a certain range (*e.g.*, 90° to -90°).

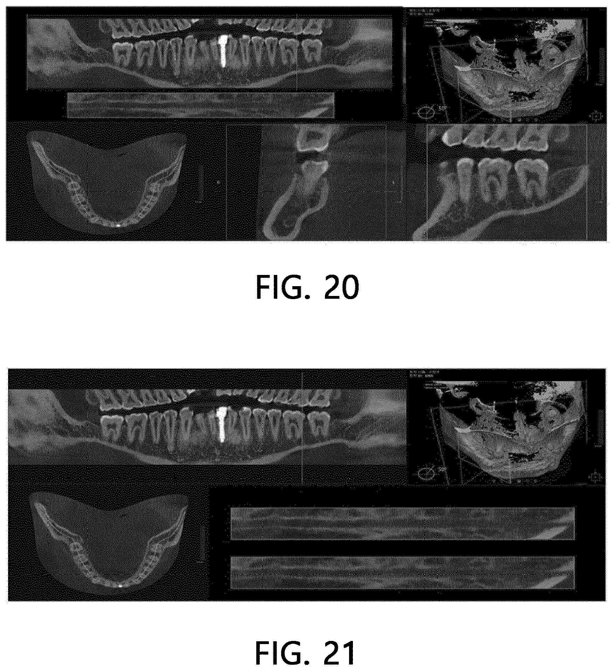

FIGS. 18 to 21 illustrate various examples of a user interface (UI) that displays curved planar reformation images.

Referring to FIGS. 18 to 21, for user convenience, a CPR image may be displayed together with a reference image. In this case, the reference image refers to a 3D image from which a CPR image is derived or an image obtained by processing the corresponding 3D image. In particular, when such a UI is provided, the CPR image and the reference image may be simultaneously displayed, but the nerve location region may be displayed in the reference image.

In addition, when the above-described UI is provided, in S500, the selection of a specific nerve among a plurality of nerves may be input from the reference image through the Iputter 120, and a CPR image may be obtained based on the input nerve location. For example, the selection for the left or right side of the inferior alveolar nerve may be input through the Iputter 120, and S500 may be performed according to the input data such that CPR images based on the corresponding nerve location (left inferior alveolar nerve location or right inferior alveolar nerve location) may be obtained.

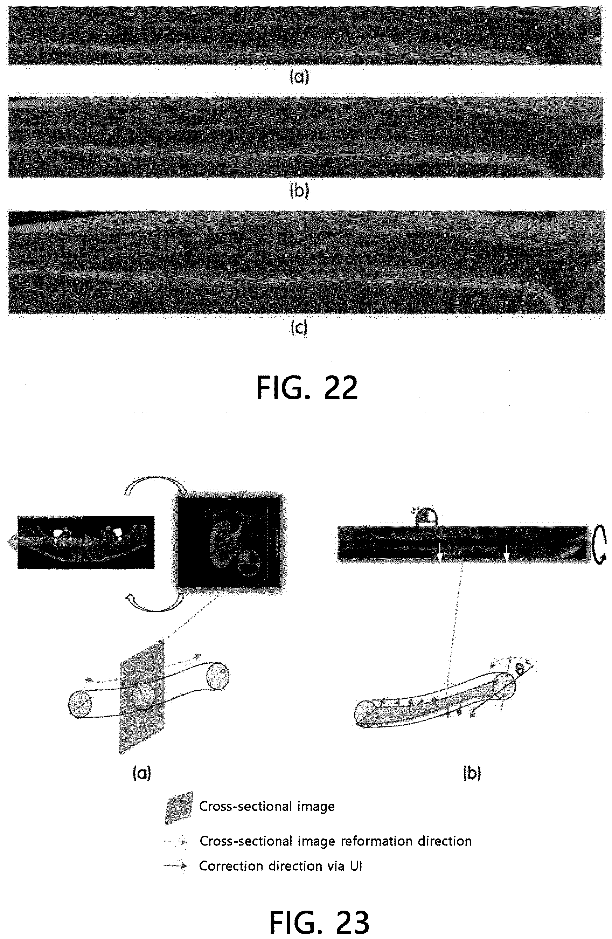

FIG. 22 illustrates various ranges of curved planar reformation images.

Also, in addition to the angle ($\Theta$) for the CPR image, a range for the CPR image may be input and CPR images reflecting the same may be obtained in S500. In this case, the range for the CPR image is a value that may be input from the user through the Iputter 120 and may be a value for r and the like. As a result, as illustrated in FIG. 22, various ranges of CPR images may be obtained. Afterwards, CPR images reflecting the corresponding range and angle ($\Theta$) may be displayed in real time in S600.

Meanwhile, when the above-described UI is provided, the angle ($\Theta$) or the range for the CPR image may be selected through the Iputter 120. For example, the angle ($\Theta$) or the range may be selected through a mouse wheel manipulation, a mouse drag input, a keyboard direction key input, a keyboard value input for providing a scroll bar on the Display 130 and the like. In particular, the angle ($\Theta$) or the range of the CPR image may increase or decrease, according to the operation of the mouse wheel or the input of the direction keys on the keyboard (input with other keys on the keyboard). Such an input means has an advantage of providing greater convenience to the user on the above-described UI.

FIG. 23 is a diagram showing a comparison between the conventional correction method and the method for correcting a nerve location according to an exemplary embodiment of the present invention.

Meanwhile, in the conventional correction method, as illustrated in FIG. 23(a), the movement and correction of the cross-sectional image are performed for the cross-sectional image of a cylinder. On the other hand, in the method for correcting a nerve location according to an exemplary embodiment of the present invention, as illustrated in FIG. 23(b), cross-sectional rotation and correction are performed on the CPR image based on the center point of a cylinder.

The exemplary embodiments of the present invention disclosed in the present specification and drawings are only provided as specific examples in order to easily explain the technical contents of the present invention and help the understanding of the present invention, and are not intended to limit the scope of the present invention.

The nerve detection method and device according to the present invention can be used in various fields of dental treatment such as implant surgery and the like.

**Claims**

1. A method for detecting a nerve, comprising:

    acquiring a learning image (S100) including a plurality of slices arranged in a coronal plane direction;
    generating learning data in which a nerve and mental tubercle and/or a mandibular angle, serving as a reference for detecting the nerve, are set in the learning image, and learning the learning data so as to generate a learning model (S200);
    inputting a target image into the learning model (S300);
    and detecting a nerve from the target image (S400) on the basis of the learning data.

2. The method of claim 1, wherein the detecting a nerve from the target image comprises:

    detecting the mental tubercle and mandibular angle from the target image;
    setting a section between the detected mental tubercle and mandibular angle as a nerve effective section; and
    detecting the nerve by searching the nerve effective section.

3. The method of claim 1, wherein the generating learning data is generating the learning data in which at least one of the alveolar bone, the mental nerve and the inferior alveolar nerve is set in the learning image.

4. The method of claim 3, wherein the detecting the nerve from the target image is detecting the alveolar bone, the mental nerve and the inferior alveolar nerve from the target image.

5. The method of claim 4, wherein the detecting the inferior alveolar nerve is detecting the inferior alveolar nerve using the detected alveolar bone.

6. The method of claim 2, wherein the detecting the mental tubercle from the target image is detecting the mental tubercle by setting a first search starting point based on statistics of the learning image, and searching in an outer direction of the target image from the first search starting point.

7. The method of claim 2, wherein the detecting the mandibular angle from the target image is detecting the mandibular angle by setting a second search starting point based on statistics of the learning image, and searching in an outer direction of the target image from the second search starting point.

8. The method of claim 1, wherein the detecting a nerve from the target image comprises:

    setting a target region expected to include the mental tubercle, the mandibular angle and the nerve which are detection targets in the target image;
    calculating location and size information of the target region; and
    calculating a probability value that the detection target is included in the target region.

9. The method of claim 8, wherein the detecting a nerve from the target image further comprises detecting the target region in which the probability value is more than or equal to a reference value as a detection region including the detection target.

10. The method of claim 9, wherein the detecting a nerve from the target image further comprises:

    extracting coordinates of a region including the nerve from the detection region; and
    detecting the nerve based on the coordinates.

11. The method of claim 1, further comprising:

    acquiring a curved planar reformation (CPR) image based on the nerve location;
    respectively displaying a nerve location and a reference line on the curved planar reformation image; and
    correcting the nerve location on the curved planar reformation image based on the reference line.

12. The method of claim 11, wherein the coordinate values of the reference line include at least one of the coordinate values of the nerve location.

13. The method of claim 11, wherein the reference line is a line passing through a part of a nerve region displayed in the curved planar reformation image.

14. A device for detecting a nerve, comprising:

    an Image collector (151) for acquiring a learning image including a plurality of slices arranged in a coronal plane direction;
    a Learner (152) for generating learning data in which a nerve and mental tubercle and/or a mandibular angle, serving as a reference for detecting the nerve, are set in the learning image, and learning the learning data so as to generate a learning model; and

a Nerve detector (153) for detecting a nerve from the target image on the basis of the learning data.

15. The device of claim 14, further comprising:
a Corrector (154) for acquiring a curved planar reformation (CPR) image based on the nerve location, displaying a reference line on the curved planar reformation image, and correcting the nerve location based on the reference line.

**Patentansprüche**

1. Ein Verfahren zum Erkennen eines Nervs, umfassend:

Erfassen eines Lernbilds (S100), das eine Vielzahl von in einer Koronalebenenrichtung angeordneten Schichten umfasst;
Erzeugen von Lerndaten, in denen ein Nerv und Tuberculum mentale und/oder ein Mandibularwinkel, die als Referenz zum Erkennen des Nervs dienen, in das Lernbild eingestellt sind, und Lernen der Lerndaten zum Erzeugen eines Lernmodells (S200);
Eingeben eines Zielbilds in das Lernmodell (S300);
und Erkennen eines Nervs anhand des Zielbilds (S400) auf der Grundlage der Lerndaten.

2. Verfahren nach Anspruch 1, wobei das Erkennen eines Nervs anhand des Zielbilds Folgendes umfasst:

Erkennen des Tuberculum mentale und Mandibularwinkels anhand des Zielbilds;
Festlegen eines Abschnitts zwischen dem erkannten Tuberculum mentale und Mandibularwinkel als einen nervenwirksamen Abschnitt; und
Erkennen des Nervs durch Durchsuchen des nervenwirksamen Abschnitts.

3. Verfahren nach Anspruch 1, wobei das Erzeugen von Lerndaten das Erzeugen von Lerndaten ist, in denen mindestens einer der Alveolarknochen, der Nervus mentalis und der Nervus alveolaris inferior im Lernbild eingestellt ist.

4. Verfahren nach Anspruch 3, wobei das Erkennen des Nervs anhand des Zielbilds das Erkennen des Alveolarknochens, des Nervus mentalis und des Nervus alveolaris inferior anhand des Zielbilds ist.

5. Verfahren nach Anspruch 4, wobei das Erkennen des Nervus alveolaris inferior das Erkennen des Nervus alveolaris inferior unter Verwendung des erkannten Alveolarknochens ist.

6. Verfahren nach Anspruch 2, wobei das Erkennen des Tuberculum mentale anhand des Zielbilds das Erkennen des Tuberculum mentale durch Festlegen eines ersten Suchstartpunkts basierend auf Statistiken des Lernbilds und Suchen in einer äußeren Richtung des Zielbilds vom ersten Suchstartpunkt aus ist.

7. Verfahren nach Anspruch 2, wobei das Erkennen des Mandibularwinkels anhand des Zielbilds das Erkennen des Mandibularwinkels durch Festlegen eines zweiten Suchstartpunkts basierend auf Statistiken des Lernbilds und das Suchen in einer äußeren Richtung des Zielbilds vom zweiten Suchstartpunkt aus ist.

8. Verfahren nach Anspruch 1, wobei das Erkennen eines Nervs anhand des Zielbilds Folgendes umfasst:

Festlegen einer Zielregion, die voraussichtlich das Tuberculum mentale, den Mandibularwinkel und den Nerv beinhaltet, die im Zielbild Erkennungsziele sind;
Berechnen von Standort- und Größeninformationen der Zielregion; und
Berechnen eines Wahrscheinlichkeitswerts, dass das Erkennungsziel in der Zielregion enthalten ist.

9. Verfahren nach Anspruch 8, wobei das Erkennen eines Nervs anhand des Zielbilds ferner das Erkennen der Zielregion umfasst, in der der Wahrscheinlichkeitswert größer oder gleich einem Referenzwert ist, als eine Erkennungsregion, die das Erkennungsziel beinhaltet.

10. Verfahren nach Anspruch 9, wobei das Erkennen eines Nervs anhand des Zielbilds ferner umfasst:

Extrahieren von Koordinaten einer Region, die den Nerv aus der Erkennungsregion enthält; und
Erkennen des Nervs basierend auf den Koordinaten.

11. Verfahren nach Anspruch 1, ferner umfassend:

Erfassen eines gekrümmten planaren Reformations-(CPR-)Bilds basierend auf der Nervenposition;
Anzeigen einer Nervenposition und einer Referenzlinie auf dem gekrümmten planaren Reformations-Bild; und
Korrigieren der Nervenposition auf dem gekrümmten planaren Reformations-Bild basierend auf der Referenzlinie.

12. Verfahren nach Anspruch 11, wobei die Koordinatenwerte der Referenzlinie mindestens einen der Koordinatenwerte der Nervenposition beinhalten.

13. Verfahren nach Anspruch 11, wobei die Referenzlinie eine Linie ist, die durch einen Teil einer Nervenregion verläuft, die im gekrümmten planaren Reformations-Bild angezeigt wird.

14. Eine Vorrichtung zum Erkennen eines Nervs, umfassend:

einen Bildsammler (151) zum Erfassen eines Lernbilds, das eine Vielzahl von in einer Koronalebenenrichtung angeordneten Schichten umfasst;
einen Lerner (152) zum Erzeugen von Lerndaten, in denen ein Nerv und Tuberculum mentale und/oder ein Mandibularwinkel, die als Referenz zum Erkennen des Nervs dienen, in das Lernbild eingestellt sind, und Lernen der Lerndaten zum Erzeugen eines Lernmodells; und
einen Nervendetektor (153) zum Erkennen eines Nervs anhand des Zielbilds basierend auf den Lerndaten.

15. Vorrichtung nach Anspruch 14, ferner umfassend:
einen Korrektor (154) zum Erfassen eines gekrümmten planaren Reformations-(CPR-)Bilds basierend auf der Nervenposition, zum Anzeigen einer Referenzlinie auf dem gekrümmten planaren Reformations-Bild und zum Korrigieren der Nervenposition basierend auf der Referenzlinie.

**Revendications**

1. Procédé pour détecter un nerf, comprenant de :

acquérir une image d'apprentissage (S100) incluant une pluralité de coupes disposées dans une direction de plan frontal ;
générer des données d'apprentissage dans lesquelles un nerf et un tubercule mentonnier et/ou un angle mandibulaire, servant de référence pour la détection du nerf, sont définis dans l'image d'apprentissage, et apprendre les données d'apprentissage de manière à générer un modèle d'apprentissage (S200) ;
entrer une image cible dans le modèle d'apprentissage (S300) ;
et détecter un nerf à partir de l'image cible (S400) sur la base des données d'apprentissage.

2. Procédé selon la revendication 1, dans lequel la détection d'un nerf à partir de l'image cible comprend de :

détecter le tubercule mentonnier et l'angle mandibulaire à partir de l'image cible ;
définir une section entre le tubercule mentonnier et l'angle mandibulaire détectés comme une section de nerf effective ; et
détecter le nerf en recherchant la section de nerf effective.

3. Procédé selon la revendication 1, dans lequel la génération de données d'apprentissage consiste à générer les données d'apprentissage dans lesquelles au moins un élément parmi l'os alvéolaire, le nerf mentonnier et le nerf alvéolaire inférieur est défini dans l'image d'apprentissage.

4. Procédé selon la revendication 3, dans lequel la détection du nerf à partir de l'image cible consiste à détecter l'os alvéolaire, le nerf mentonnier et le nerf alvéolaire inférieur à partir de l'image cible.

5. Procédé selon la revendication 4, dans lequel la détection du nerf alvéolaire inférieur consiste à détecter le nerf alvéolaire inférieur en utilisant l'os alvéolaire détecté.

6. Procédé selon la revendication 2, dans lequel la détection du tubercule mentonnier à partir de l'image cible consiste à détecter le tubercule mentonnier en définissant un premier point de début de recherche sur la base de statistiques de l'image d'apprentissage, et en effectuant une recherche dans une direction extérieure de l'image cible à partir du premier point de début de la recherche.

7. Procédé selon la revendication 2, dans lequel la détection de l'angle mandibulaire à partir de l'image cible consiste à détecter l'angle mandibulaire en définissant un second point de début de recherche sur la base de statistiques de l'image d'apprentissage, et en effectuant une recherche dans une direction extérieure de l'image cible à partir du second point de début de recherche.

8. Procédé selon la revendication 1, dans lequel la détection d'un nerf à partir de l'image cible comprend de :

définir une région cible qui devrait inclure le tubercule mentonnier, l'angle mandibulaire et le nerf, qui sont des cibles de détection dans l'image cible ;
calculer des informations d'emplacement et de taille de la région cible ; et
calculer une valeur de probabilité que la cible de détection soit incluse dans la région cible.

9. Procédé selon la revendication 8, dans lequel la détection d'un nerf à partir de l'image cible comprend en outre la détection de la région cible dans laquelle la valeur de probabilité est supérieure ou égale à une valeur de référence, comme une région de détection incluant la cible de détection.

10. Procédé selon la revendication 9, dans lequel la détection d'un nerf à partir de l'image cible comprend en outre de :

extraire de la région de détection des coordonnées d'une région incluant le nerf ; et
détecter le nerf sur la base des coordonnées.

11. Procédé selon la revendication 1, comprenant en outre de :

acquérir une image de reconstruction planaire curviligne (CPR) basée sur l'emplacement de nerf ;
afficher respectivement un emplacement de nerf et une ligne de référence sur l'image de reconstruction planaire curviligne ; et
corriger l'emplacement de nerf sur l'image de reconstruction planaire curviligne basée sur la ligne de référence.

12. Procédé selon la revendication 11, dans lequel les valeurs de coordonnées de la ligne de référence incluent au moins l'une des valeurs de coordonnées de l'emplacement de nerf.

13. Procédé selon la revendication 11, dans lequel la ligne de référence est une ligne passant par une partie d'une région de nerf affichée dans l'image de reconstruction planaire curviligne.

14. Dispositif pour détecter un nerf, comprenant :

un collecteur d'image (151) pour acquérir une image d'apprentissage incluant une pluralité de coupes disposées dans une direction de plan frontal ;
un système d'apprentissage (152) pour générer des données d'apprentissage dans lesquelles un nerf et un tubercule mentonnier et/ou un angle mandibulaire, servant de référence pour détecter le nerf, sont définis dans l'image d'apprentissage, et apprendre les données d'apprentissage de manière à générer un modèle d'apprentissage ; et
un détecteur de nerf (153) pour détecter un nerf à partir de l'image cible sur la base des données d'apprentissage.

15. Dispositif selon la revendication 14, comprenant en outre :
un correcteur (154) pour acquérir une image de reconstruction planaire curviligne (CPR) basée sur l'emplacement de nerf, afficher une ligne de référence sur l'image de reconstruction planaire curviligne, et corriger l'emplacement de nerf sur la base de la ligne de référence.

100

110

Communicator

150

Controller

130

Display

Inputter

Memory

120

140

# FIG. 1

Image collector —151

Learner —152

Nerve detector —153

Corrector —154

# FIG. 2

Acquire learning image —S100

Generate learning data and learning model —S200

Input target image into learning model —S300

Detect nerve in target image —S400

FIG. 3

(a) (b) (c)

FIG. 4

FIG. 5

FIG. 6

Detect mental tubercle and
mandibular angle from target image ———S410

Set the section between mental tubercle and
mandibular angle as nerve effective section ———S420

Detect nerve by searching the
nerve effective section ———S430

# FIG. 7

FIG. 8

Set target region in target image ——S431

Calculate location information
of target region ——S432

Calculate probability value that target
region is detection target ——S433

Detect target region whose probability
value is more than or equal to
reference value as detection region ——S434

Extract center coordinates
of nerve region ——S435

Detect nerve based on
center coordinates ——S436

# FIG. 9

Acquire curved planar
reformation image ——S10

Display image ——S20

Correct nerve location ——S30

# FIG. 10

FIG. 11

FIG. 12

(a)

$C_i(\ i = 0,1,...N-1)$     $C = \{C_i \in R^3 | i = 0,1,...N-1\}$

(b)

$C_i$

$n_i = C_{i+1} - C_i$

$P_i$

(c)

$S$

$\widehat{r_i}$

$C_i$

$\vec{r}$

$\vec{n_i}$

$P_i$

(d)

$S$

$r_i$

$r$

$\theta$

$v_i$   $P_i$

(e)

$C_0 + kv_0$     $C_{N-1} + kv_{N-1}$

$S$

$C_i + kv_i$

(f)

$S$

$\theta$

CPR

# FIG. 13

FIG. 14

FIG. 15

FIG. 16

FIG. 17

FIG. 18

FIG. 19

FIG. 20

FIG. 21

(a)

(b)

(c)

# FIG. 22

(a)

(b)

Cross-sectional image

Cross-sectional image reformation direction

Correction direction via UI

# FIG. 23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019002631 A1